(19) Europäisches Patentamt
European Patent Office
Office européen des brevets

(11) Veröffentlichungsnummer: **0 495 222 A1**

(12) **EUROPÄISCHE PATENTANMELDUNG**

(21) Anmeldenummer: **91121768.5**

(51) Int. Cl.$^5$: **C07C 317/14**, C07C 315/00

(22) Anmeldetag: **19.12.91**

(30) Priorität: **15.01.91 DE 4100976**

(43) Veröffentlichungstag der Anmeldung:
**22.07.92 Patentblatt 92/30**

(84) Benannte Vertragsstaaten:
**BE DE FR GB NL**

(71) Anmelder: **BASF Aktiengesellschaft**
**Carl-Bosch-Strasse 38**
**W-6700 Ludwigshafen(DE)**

(72) Erfinder: **Hupfeld, Bernd, Dr.**
**Mausbergweg 6**
**W-6720 Speyer(DE)**
Erfinder: **Reichelt, Helmut, Dr.**
**Johann-Gottlieb-Fichte-Strasse 56**
**W-6730 Neustadt(DE)**

(54) **Verfahren zur Herstellung von Biphenylderivaten durch Umsetzung von Biphenyl mit Säurehalogeniden.**

(57) Verfahren zur Herstellung von Verbindungen der allgemeinen Formel I

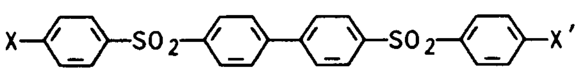 I,

wobei X und X' unabhängig voneinander F oder Cl sind, durch Umsetzung von Biphenyl mit Verbindungen der allgemeinen Formeln II und/oder III

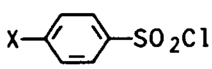 II

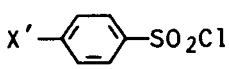 III,

wobei X und X' die oben angegebene Bedeutung haben, in Gegenwart einer Lewis-Säure, wobei man Biphenyl und die Monomeren der Formel II und/oder III im Molverhältnis von 1:2,5 bis 1:4 umsetzt und die Umsetzung in Abwesenheit von Lösungsmitteln durchführt.

EP 0 495 222 A1

Die Erfindung betrifft ein Verfahren zur Herstellung von Verbindungen der allgemeinen Formel I

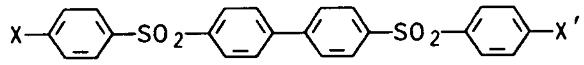
I,

wobei X und X' unabhängig voneinander F oder Cl sind, durch Umsetzung von Biphenyl mit Verbindungen der allgemeinen Formeln II und/oder III

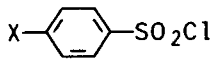
II

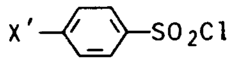
III,

wobei X und X' die oben angegebene Bedeutung haben, in Gegenwart einer Lewis-Säure.

Verbindungen der allgemeinen Formel I sind für die Herstellung von hochtemperaturbeständigen Thermoplasten (Polyarylethersulfonen) von Interesse. Um gute Polymereigenschaften zu erzielen, müssen die eingesetzten Monomeren einen hohen Reinheitsgrad aufweisen.

Aus Soc.Plast.Eng.Nat.Conf.Techn.Papers (1975), Seiten 621-623 ist ein Verfahren zur Herstellung von 4,4'-Bis-(4-chlorphenylsulfonyl)-biphenyl bekannt, nach welchem Biphenyl und 4-Chlorbenzolsulfonsäurechlorid im Molverhältnis 1:2,25 in Anwesenheit von 1 bis 10 Gew.%, bezogen auf die Ausgangsstoffe, Fe-(III)chlorid bei einer Temperatur von 140 °C umgesetzt werden. Die Umsetzung in Substanz führt zu einer Ausbeute von 65 %, bei Verwendung von Nitrobenzol als Lösungsmittel konnten Ausbeuten von etwa 80 % erzielt werden. Für die Herstellung von 4,4'-Bis-(4-chlorphenylcarbonyl)-biphenyl ist die analoge Reaktion in der DE-A 24 25 199 beschrieben.

Nachteilig bei diesem Verfahren ist die hohe Toxizität des Nitrobenzols, die Gefahr explosionsartiger Reaktionen des $FeCl_3$ mit dem Nitrobenzol und der nicht zufriedenstellende Reinheitsgrad (der am Schmelzpunkt von 271 bis 273 °C erkennbar ist).

In der US-A-4 303 776 wird die Umsetzung von Biphenyl mit 4-Chlorbenzolsulfonsäurechlorid in Anwesenehit von $FeCl_3$ im Molverhältnis 1:2,25 bei 140 °C beschrieben. Die Ausbeute betrug 45 % eines Produkts mit einem Schmelzpunkt von 269 bis 272 °C.

Aufgabe der vorliegenden Erfindung war es daher, ein verbessertes Verfahren zur Herstellung von Monomeren der allgemeinen Formel I zur Verfügung zu stellen, welches die vorstehend geschilderten Nachteile nicht aufweist, insbesondere in hoher Ausbeute zu Produkten mit hoher Reinheit führt.

Diese Aufgabe wurde erfindungsgemäß durch ein Verfahren gemäß Anspruch 1 gelöst, wobei Biphenyl und die Monomeren der Formel II und/oder III im Molverhältnis von 1:2,5 bis 1:4 eingesetzt werden und die Umsetzung in Abwesenheit von Lösungsmitteln durchgeführt wird.

Dadurch werden sehr reine Produkte in hoher Ausbeute erhalten.

Bevorzugte Ausführungsformen des Verfahrens sind den Unteransprüchen zu entnehmen.

Nach dem erfindungsgemäßen Verfahren können folgende Verbindungen hergestellt werden:
4,4'-Bis-(4-chlorphenylsulfonyl)-biphenyl (I1),
4,4'-Bis-(4-fluorphenylsulfonyl)-biphenyl (I2),
[4-(4-Chlorphenylsulfonyl)-4'-(4-fluorphenylsulfonyl)]-biphenyl (I3),

Diese können mit Hilfe der Parameter in Formel I folgendermaßen dargestellt werden:

| | X | X' |
|---|---|---|
| I1 | Cl | Cl |
| I2 | F | F |
| I3 | Cl | F |

Die Herstellung dieser Verbindungen erfolgt nach dem erfindungsgemäßen Verfahren durch Umsetzung von Biphenyl mit mindestens einer Verbindung der allgemeinen Formeln II oder III, d.h. mit 4-Chlor- bzw. 4-Fluorbenzolsulfonsäurechlorid. Diese Ausgangsstoffe sind im Handel erhältlich oder nach dem Fachmann bekannten Verfahren herstellbar, so daß sich hier nähere Angaben erübrigen.

Bei der Herstellung der Verbindungen I3 wird die Umsetzung vorteilhafterweise in zwei Stufen durchgeführt, wobei zunächst Biphenyl mit 4-Chlorbenzolsulfonsäurechlorid bzw. 4-Chlorbenzoylchlorid umgesetzt und anschließend die Umsetzung mit der Fluorverbindung vorgenommen wird.

Bevorzugte Ausgangsverbindung ist 4-Chlorphenylsulfonylchlorid.

Das Molverhältnis von Verbindungen der allgemeinen Formel II und/oder III zu Biphenyl liegt im Bereich von 2,5 bis 4:1, insbesondere von 2,8:1 bis 4:1.

Die Reaktion wird in Gegenwart einer Lewis-Säure als Katalysator durchgeführt. Geeignete Lewis-Säuren als Katalysatoren für Friedel-Crafts-Acylierungen sind dem Fachmann bekannt und in der Literatur beschrieben. Ganz allgemein sind darunter Verbindungen zu verstehen, die als Elektronenpaarakzeptor fungieren können. Nur beispielsweise seien hier $AlCl_3$, $AlBr_3$, $SbCl_5$, $BCl_3$, $BF_3$, $FeCl_3$, $SnCl_2$, $ZnCl_2$, $TiCl_4$ und $MoCl_5$ genannt, um nur einige zu nennen. Von diesen wird $FeCl_3$ besonders bevorzugt.

Die eingesetzte Menge an Lewis-Säure kann abhängig von der Art der gewählten Lewis-Säure, in weiten Grenzen variieren, vorzugsweise werden aber Biphenyl und Lewis-Säure im Molverhältnis von 100:1 bis 10:1 eingesetzt, d.h. die Lewis-Säure wird in Mengen von 1 bis 10 mol-%, bezogen auf Biphenyl , eingesetzt.

Aus dem Vorstehenden geht hervor, daß eine besonders bevorzugte Variante des erfindungsgemäßen Verfahrens die Umsetzung von Biphenyl mit 4-Chlorphenylsulfonylchlorid in Anwesenheit von $FeCl_3$ ist.

Die Umsetzungstemperatur beim erfindungsgemäßen Verfahren liegt im allgemeinen im Bereich von 80 bis 200, vorzugsweise von 80 bis 180° C und insbesondere bei 100 bis 170° C. Zur Umsetzung werden bevorzugt das Sulfonsäurechlorid und der Katalysator vorgelegt und auf 100 bis 120° C aufgeheizt. In die so entstandene Schmelze wird dann das Biphenyl, das vorher verflüssigt wurde, zugetropft. Während des Zutropfens und im Anschluß daraus wird die Mischung auf die gewünschte Reaktionstemeratur erhitzt.

Die Reaktionsdauer hängt von der Reaktionstemperatur ab; das Ende der Reaktion läßt sich am Ende der Halogenwasserstoff-Entwicklung erkennen. Im allgemeinen liegt die Reaktionszeit im Bereich von 0,5 bis 6, insbesondere 1 bis 3 Stunden.

Nach Beendigung der Umsetzuzng läßt man die noch heiße Schmelze vorzugsweise in einen aliphatischen Alkohol wie Methanol, Ethanol, n-Propanol, i-Propanol oder n-Butanol einlaufen oder gibt umgekehrt den Alkohol zur noch heißen Schmelze zu. Bei beiden Verfahrensweisen hat sich Isopropanol (i-Propanol) als besonders geeignet erwiesen.

In beiden Fällen erhält man auf diese Weise eine Suspension der Verbindung der Formel I in dem entsprechenden Alkohol, die man dann abkühlen läßt.

Anschließend kann das Produkt in an sich bekannter Weise z.B. durch Filtration abgetrennt und gereinigt (z.B. durch Waschen mit Alkohol) werden, bevor es getrocknet wird.

Die nach diesem Verfahren erhältlichen Ausbeuten betragen im allgemeinen mehr als 80 % der Theorie, in den meisten Fällen werden Ausbeuten von etwa 88 bis etwa 96 % der Theorie erzielt.

Der Reinheitsgrad liegt im allgemeinen von 95 bis 99,9, besondere von 97 bis 99 Gew.-%; dieser Reinheitsgrad kann durch Umkristallisation aus Chlorbenzol, 1,2-Dichlorbenzol, Butandiol-1,2 oder insbesondere N,N-Dimethylformamid noch weiter erhöht werden.

Der Vorteil des erfindungsgemäßen Verfahrens liegt darin, daß ohne den Einsatz von zusätzlichen Lösungsmitteln Verbindungen der Formel I in hohen Ausbeuten mit hoher Reinheit erhalten werden.

Beispiel 1

In einem 5-1 Kessel aus Hastelloy C 4 wurden bei Raumtemperatur unter Stickstoffatmosphäre 2110 g (10 mol) 4-Chlorbenzolsulfonsäurechlorid und 10 g (0,075 mol) Eisen-III-chlorid vorgelegt und auf 120° C erwärmt. In die Schmelze wurden 385 g (2,5 mol) geschmolzenes Biphenyl zugetropft. Während des Zutropfens wurde die Temperatur im Kessel auf 140° C erhöht, nach dem Zutropfen des Biphenyls erhöhte man die Temperatur der Reaktionslösung auf 160° C und rührte so lange nach, bis die Chlorwasserstoff-Entwicklung beendet war (Dauer ca. 1 Stunde). Die entstandene Schmelze ließ man anschließend in 2,5 l Isopropanol unter Rühren einlaufen und kühlte auf Raumtemperatur ab. Nach Filtration, Waschen mit Isopropanol und Trocknen erhielt man 1124 g (89,3 % d. Th.) 4,4'-Bis-(4-chlorphenylsulfonyl)-biphenyl in einer Reinheit von 98,34 % (durch HPLC bestimmt) und einem Schmelzpunkt von 276° C bis 277° C.

Beispiel 2

Man verfuhr wie in Beispiel 1 beschrieben, jedoch wurden 1585 g (7,5 mol) 4-Chlorbenzolsulfonsäurechlorid eingesetzt. Nach beendeter Umsetzung ließ man 2,0 l Isopropanol in die entstandene Schmelze einlaufen und auf Raumtemperatur abkühlen. Nach Filtration, Waschen und Trocknung erhielt man 1151,5 g

(91,5 % d. Th.) 4,4'-Bis-(4-chlorphenylsulfonyl)-biphenyl in einer Reinheit von 98,81 % (HPLC) und einem Schmelzpunkt von 277 ° C bis 278 ° C.

Beispiel 3

Reinigung von 4,4'-Bis-(4-chlorphenylsulfonyl)-biphenyl

1000 g der wie nach Beispiel 2 hergestellten Verbindung wurden in 2,0 l N,N-Dimethylformamid suspendiert und auf 145 ° C erwärmt, bis eine klare Lösung entstanden war (Dauer ca. 10 Minuten). Anschließend ließ man unter Rühren auf Raumtemperatur abkühlen. Nach Filtration, Waschen mit Isopropanol und Trocknung erhielt man 817 g (81,7 % d. Th.) reines 4,4'-Bis-(4-chlorphenylsulfonyl)-biphenyl in einer Reinheit von 99,82 % (HPLC) und einem Schmelzpunkt von 279 ° C bis 280 ° C.

**Patentansprüche**

**1.** Verfahren zur Herstellung von Verbindungen der allgemeinen Formel I

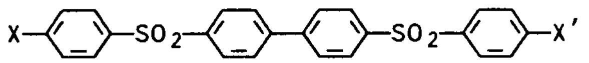

I,

wobei X und X' unabhängig voneinander F oder Cl sind, durch Umsetzung von Biphenyl mit Verbindungen der allgemeinen Formeln II und/oder III

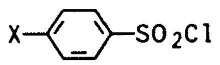

II

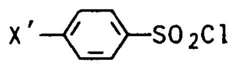

III,

wobei X und X' die oben angegebene Bedeutung haben, in Gegenwart einer Lewis-Säure, dadurch gekennzeichnet, daß man Biphenyl und die Verbindungen der Formeln II und/oder III im Molverhältnis 1:2,5 bis 1:4 einsetzt und die Umsetzung in Abwesenheit von Lösungsmitteln durchführt.

**2.** Verfahren nach Anspruch 1, dadurch gekennzeichnet, daß das Molverhältnis von Biphenyl zur Lewis-Säure im Bereich von 1:0,01 bis 1:0,1 liegt.

**3.** Verfahren nach einem der Ansprüche 1 oder 2, dadurch gekennzeichnet, daß man Biphenyl mit 4-Chlorbenzolsulfonsäurechlorid umsetzt.

Europäisches Patentamt

# EUROPÄISCHER RECHERCHENBERICHT

Nummer der Anmeldung

EP 91 12 1768

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | SOCIETY OF PLASTIC ENGINEERS, TECHNICAL PAPERS Bd. 21, 1975, Seiten 621 - 623; R.J. CORNELL ET AL.: 'A NEW POLYARYL SULFONE THERMOPLASTIC RETAINING MECHANICAL PROPERTIES UP TO 400 F' * Seite 621, Spalte 1, Zeile 48 - Spalte 2, Zeile 33 * | 1-3 | C07C317/14<br>C07C315/00 |
| Y | EP-A-0 351 954 (AMOCO CORPORATION) * Seite 11 - Seite 12; Ansprüche 1-3,5 * * Seite 4, Zeile 54 - Zeile 56 * * Seite 5, Zeile 11 - Zeile 15 * * Seite 8 - Seite 9; Beispiele 2,3 * | 1-3 | |
| D,A | US-A-4 303 776 (A.L. BARON ET AL.) * Spalte 4, Zeile 7 - Zeile 20 * | 1-3 | |
| A | EP-A-0 233 409 (TOA NENRYO KOGYO KABUSHIKI KAISHA) * Seite 9, Zeile 12 - Seite 10, Zeile 3 * * Seite 19; Beispiel 2 * | 1-3 | **RECHERCHIERTE SACHGEBIETE (Int. Cl.5)**<br>C07C |

Der vorliegende Recherchenbericht wurde für alle Patentansprüche erstellt

| Recherchenort | Abschlußdatum der Recherche | Prüfer |
|---|---|---|
| DEN HAAG | 26 MAI 1992 | FINK D.G. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)